(19) Europäisches Patentamt
European Patent Office
Office européen des brevets

(11) EP 1 126 026 A1

(12) **EUROPEAN PATENT APPLICATION**
published in accordance with Art. 158(3) EPC

(43) Date of publication:
**22.08.2001 Bulletin 2001/34**

(21) Application number: **99951123.1**

(22) Date of filing: **28.10.1999**

(51) Int Cl.[7]: **C12N 15/11**, C12N 15/63, C12N 5/10, C12P 21/02, C12N 9/02

(86) International application number:
**PCT/JP99/05984**

(87) International publication number:
**WO 00/26358 (11.05.2000 Gazette 2000/19)**

(84) Designated Contracting States:
**AT BE CH CY DE DK ES FI FR GB GR IE IT LI LU MC NL PT SE**

(30) Priority: **30.10.1998 JP 31042298**
**16.11.1998 JP 32534498**

(71) Applicant: **Medical & Biological Laboratories Co., Ltd.**
**Nagayo-shi, Aichi 460-0002 (JP)**

(72) Inventors:
- **TOJI, Shingo Medical & Biological Lab. Co. Ltd.**
  **Ina-shi Nagano 396-0002 (JP)**
- **YANO, Minoru Medical & Biological Lab. Co. Ltd.**
  **Ina-shi Nagano 396-0002 (JP)**
- **TAMAI, Katsuyuki**
  **Medical & Biological Lab. Co.Ltd.**
  **Ina-shi Nagano 396-0002 (JP)**

(74) Representative: **VOSSIUS & PARTNER**
**Siebertstrasse 4**
**81675 München (DE)**

(54) **SELENOCYSTEINE INSERTION SEQUENCE**

(57) A novel selenocysteine insertion sequence (SECIS) is described. The SECIS of this invention, which consists of less than 360 b, ensures the insertion of selenocysteine into a translational region. It is useful in expressing selenoproteins having selenocysteine as an essential constituting amino acid, such as thioredoxin reductase.

EP 1 126 026 A1

## Description

### Technical Field

The present invention relates to a nucleotide sequence referred to as selenocysteine insertion sequence (abbreviated as SECIS hereinafter).

### Background Art

Selenoprotein is a general term for proteins containing selenium (Se), one of the essential trace elements, as the selenocysteine (SeCys) residue. Selenocysteine has a structure such that Se is the constituting element, replacing the S (sulfur) component of cysteine. SeCys has been reported to have radioprotective and anticancer actions. SeCys-containing proteins, such as glutathione peroxidase (GPx), which glutathione- or thioredoxin- dependently reduces an active oxygen species hydroperoxide (-OOH) to eliminate it, type I tetraiodothyronine deiodinase, which converts the thyroid hormone (thyroxine) precursor T4 into the active type T3, selenoprotein P, containing 10 selenocysteine residues, and selenoprotein W, a low molecular weight SeCys-containing protein present in muscles, have hitherto been reported. The functions of selenoprotein P and selenoprotein W have not been fully elucidated. In addition, human thioredoxin reductase (abbreviated as TxR hereinafter) has been reported to contain SeCys in the amino acid sequence Cys-SeCys-Gly-termination codon (TAA). In the most well-studied bovine TxR, it has been reported that the C-terminal SeCys is essential for the expression of the enzyme activity since the carboxypeptidase Y treatment results in the release of the C-terminal SeCys with a concomitant loss of the enzyme activity (Zhong, L., E. S. Arn-er, et al. (1998). Rat and calf thioredoxin reductase are homologous to glutathione reductase with a carboxyl-terminal elongation containing a conserved catalytically active penultimate selenocysteine residue. J. Biol. Chem. 273 (15): 8581-8591). Therefore, selenocysteine is thought to have a tremendous impact on the activity of selenoproteins such as TxR.

Interestingly, SeCys is the twenty first translatable amino acid encoded by the termination codon UGA in its unique biosynthetic system. Furthermore, it has been elucidated that the presence of not only the translational region but also the 3'-terminal untranslated region (abbreviated as 3'UTR hereinafter), located downstream of the above translational region, is important to translate UGA into selenocysteine instead of recognizing it as the termination codon (Marla J. et al. Recognition of UGA as a selenocysteine codon in type I deiodinase requires sequences in the 3' untranslated region, Nature 353, 273-276, 1991; Kristina E. Hill et al. Conserved nucleotide sequence in the open reading flame and 3' untranslated region of selenoprotein P mRNA. Proc. Natl. Acad. Sci. USA, 90, 537-541, 1993; Marla J. et al. Functional characterization of the eukaryotic SECIS elements which direct selenocysteine insertion at UGA codons. EMBO J., 12, 3315-3322, 1993; Thressa C. Standtman, Selenocysteine, Annu. Rev. Biochemistry, 65, 83-100, 1996).

In humans, TxR has been proved to be one of the selenoproteins. Like bovine TxR, human TxR contains SeCys as a constituting amino acid in its C-terminus, which is also assumed to play an important role in the enzymatic activity. In addition, it has been proved that the region corresponding to the above-described SECIS is present in the 3'UTR of the cDNA thereof (Pamera Y. et al., Cloning and sequencing of a human thioredoxin reductase., FEBS Letters, 373, 5-9, 1995; Koishi R. et al., Cloning and characterization of a novel oxidreductase KDRF from human bone marrow-derived stromal cell Line KM-102, J. Biol. Chem., 272, 2570-2577, 1997).

From the information obtained hitherto, it is thought that a stem-loop structure is formed in the 3'UTR, consisting of several thousand nucleotides, which makes the UGA codon present in the translational region to be recognized as the selenocysteine codon but not as a termination codon, and induces the incorporation of selenocysteine into the protein. A region essential for inserting selenocysteine into the translational region is the nucleotide sequence referred to as a "SECIS". Thus, it would be understood that the expression of a protein having an enzymatic activity that is greatly influenced by selenocysteine must be accompanied by a SECIS in the 3'UTR.

However, known SECISs have an essential region sequence that is up to several thousand nucleotides long. Such long nucleotide sequences may become a hindrance to a vector having limited capacity for insertion of nucleotide sequence. Furthermore, the increase in size of cDNA, including the transcriptional region as a whole, corresponds to a decrease the amplification efficiency by PCR, and/or an increase in insert mutations during the amplification by PCR. Therefore, there is a demand in the art for a novel SECIS comprising as few constituting nucleotides as possible, and yet capable of inserting selenocysteine in the translational region without fail.

### Disclosure of the Invention

It is an object of the present invention to provide a novel SECIS, selenoprotein expression vectors including the SECIS, and uses thereof.

Present inventors have isolated cDNAs encoding novel proteins, namely human thioredoxin reductase IIα and IIβ (abbreviated as TxRIIα and TxRIIβ respectively, and TxRIIs representing both, hereinafter) , from human placenta cDNA libraries, and filed it as the patent application Japanese Patent Application No. Hei 10-310422. In the process for screening factors binding to one of inhibitors of apoptosis protein in humans, XIAP (X-linked inhibitor of apoptosis protein), genes encoding these novel proteins were isolated. Present inventors have analyzed the putative amino acid sequence from cDNA for the protein, and discovered the presence of the same amino acid sequence Cys-SeCys-Gly-termination codon (TAA) as that in the known TxR near the C-terminus (SEQ ID NOs: 3 and 5). Furthermore, they have clarified the SECIS which brings about the insertion of this selenocysteine by comparing it with SECISs reported for other selenoproteins described above and 3'UTR of TxRIIα to accomplish this invention. Thus, the present invention provides the following SECIS, selenoprotein expression vectors using the SECIS, and uses thereof:

[1] a DNA comprising the nucleotide sequence as defined in SEQ ID NO: 1;
[2] a DNA comprising the nucleotide sequence as defined in SEQ ID NO: 1, wherein one or more nucleotides are substituted, deleted, added, and/or inserted, that inserts selenocysteine for the TGA codon contained in the translational region located upstream of said DNA;
[3] a DNA that hybridizes under stringent conditions to a DNA having the nucleotide sequence as defined in SEQ ID NO: 1, and that inserts selenocysteine for the TGA codon contained in the translational region located upstream of said DNA;
[4] a DNA that hybridizes to the nucleotide sequence as defined in SEQ ID NO: 1 and that has a chain length of at least 15 nucleotides;
[5] a selenoprotein expression vector comprising the following elements:

a) a cloning site, to which a DNA encoding a selenoprotein amino acid sequence can be inserted,
b) the DNA of any one of [1], [2], and [3] ligated downstream of said cloning site, and
c) a regulatory region required for protein expression;

[6] the selenoprotein expression vector of [5], further comprising a DNA encoding a selenoprotein amino acid sequence inserted at the cloning site ;
[7] a transformant transformed with the selenoprotein expression vector of [6];
[8] a method for preparing a selenoprotein comprising the steps of culturing a transformant of [7] and collecting the selenoprotein;
[9] a method for preparing a selenoprotein of [8], wherein the selenoprotein is an enzyme containing a selenocysteine in its active center;
[10] the method for preparing a selenoprotein of [9], wherein said enzyme containing selenocysteine in its active center is thioredoxin reductase;
[11] a DNA that is antisense to the DNA of [1] or a portion thereof; and
[12] a method of screening for substances that regulate the expression of selenoproteins, comprising the steps of:

a) bringing candidate compounds into contact with the transformant of [7],
b) measuring the selenoprotein contained in the host cells or the culture supernatant thereof and comparing it with controls, and
c) selecting a compound that alters the expression level of selenoproteins by comparison with controls.

SECIS of the present invention can be chemically synthesized according to the nucleotide sequence as defined in SEQ ID NO: 1, or can be obtained from cDNAs of human TxRIIα or TxRIIβ. These cDNAs are contained in the Human Placenta MATCHMAKER cDNA library, a commercial placenta cDNA library from CLONTECH. Therefore, it is easy to obtain a required region by performing PCR, using primers enabling specific amplification of the required region. Nucleotide sequences of human TxRIIα and TxRIIβ cDNA are set forth in SEQ ID NOs: 2 and 4.

Certain variations in the nucleotide sequence of SECIS are also included in the present invention, inasmuch as these modified SECISs are capable of inserting selenocysteine for the TGA codon to be translated into selenocysteine, which is contained in the translational region of selenoprotein located upstream of the SECIS. Specifically, the stem-loop structure (Fig. 4) formed by the mRNA which is transcribed, based on the DNA set forth in SEQ ID NO: 1, bears a critical role in the selenocysteine insertion. Therefore, variations in the nucleotide sequence that do not alter this structure a great deal are included in this invention. For example, DNAs comprising the nucleotide sequence according to SEQ ID NO: 1, wherein one or more nucleotides are substituted, deleted, added, and/or inserted, that are capable of inserting selenocysteine for the TGA codon contained in the translational region located upstream of the DNA are included in this invention. In addition, DNAs that hybridize under stringent conditions to a DNA comprising the nucleotide sequence set forth in SEQ ID NO: 1, and that are capable of inserting selenocysteine for the TGA codon contained in the translational region located upstream of the DNA are also included in this invention. Accordingly, SECIS homologues derived from species other than humans are included in the SECIS of this invention.

Many of nucleotide sequences capable of hybridizing under stringent conditions to a specific sequence are thought to have similar activities to the functions borne by the specific sequence. An exemplary set of hybridization condition is as follows: , in 5x SSC at 25°C in the absence of formamide, preferably in 6x SSC, 40% formamide at 25°C, more preferably in 5x SSC, 50% formamide at 40°C. Washing after hybridization is carried out, for example, in 2x SSC at 37°C, preferably in lx SSC at 55°C, and more preferably in lx SSC at 60°C.

Whether a DNA with a certain nucleotide sequence is capable of inserting selenocysteine for the TGA codon contained in the translational region located upstream of the DNA can be ascertained by examining the amino acid sequence of expression product of an expression vector inserted with the DNA downstream of the DNA encoding selenoprotein. Namely, the translation of the DNA into a protein with the expected molecular weight is considered as a proof that the insertion of selenocysteine is achieved. Alternatively, when selenocysteine constitutes the active center, the insertion thereof can be confirmed by examining the activity of the protein. TXRIIs, and such, are used, for example, as selenoproteins.

By ligating SECIS thus obtained downstream of a cDNA encoding a selenoprotein beforehand, the SECIS inserts selenocysteine for the UGA codon in the translation of the sequence. SECIS of this invention may be positioned at any position relative to the translational region. That is, SECIS can be ligated or inserted to any arbitrary sites of the 3'UTR of the former. However, since one of advantages of this invention is its ability to shorten the whole cDNA including the 3'UTR, it is preferable not to leave unnecessary 3'UTR. Furthermore, the efficiency of selenocysteine uptake can be controlled by relocating the stem-loop structure. For example, in the chimeric gene for type I tetraiodothyronine deiodinase, a 4-fold elevation of selenocysteine insertion activity has been observed, as compared with that by the original cDNA, by ligating the other SECIS of selenoprotein than the original one to the 3'UTR (Maria J. et al. Functional characterization of the eukaryotic SECIS elements which direct selenocysteine insertion at UGA codons. EMBO J., 12, 3315-3322, 1993). Based on these prior arts, one can locate the SECIS of this invention so as to achieve efficient insertion of selenocysteine.

SECIS of the present invention can be incorporated beforehand, together with a regulatory region required for the protein expression, into an expression vector to form a selenoprotein expression vector. A vector expressing a selenoprotein in its complete form, containing selenocysteine, can be easily constructed by locating the cloning site upstream of SECIS in the vector and inserting cDNA encoding the selenoprotein amino acid sequence at this site. Prior reports have demonstrated that SECISs can function even if the gene located upstream thereof is replaced with a gene for other selenoprotein (Marla J. et al. Recognition of UGA as a selenocysteine codon in type I deiodinase requires sequences in the 3' untranslated region, Nature 353, 273-276, 1991; Marla J. et al. Functional characterization of the eukaryotic SECIS elements which direct selenocysteine insertion at UGA codons. EMBO J., 12, 3315-3322, 1993). As shown in the Examples, the SECIS of the present invention has the ability to insert selenocysteine not only into the TxRIIα and TxRIIβ from which it was derived but also into other selenoproteins. That is, SECIS of this invention manifest the activity necessary to insert selenocysteine in the expression of selenoproteins in host animal cells, regardless of the kind of the proteins. Herein, the "regulatory region" required for protein expression refers to promoters and enhancers.

In the bovine TxR, it has been demonstrated that selenocysteine bears an important role in its reducing activity. Since human TxR and TxRIIs also have a structure supporting the reducing activity of bovine TxR, that is, an amino acid sequence comprising Cys-SeCys-Gly-termination codon, selenocysteine is thought to exert great effects on their enzyme activities as well. SECIS is a unique nucleotide sequence required for the translation of this selenocysteine, and an important element in the expression of these proteins.

Furthermore, the present invention also provides DNAs capable of hybridizing to the DNA set forth in SEQ ID NO: 1 and having a chain length of 15 nucleotides or more. These DNAs according to this invention are useful as probes for SECIS detection or primers for SECIS amplification. For example, the detection of mRNA which hybridizes to SECIS-specific probes in certain tissues or cultured cells indicates a possibility for the strong expression of a gene encoding a protein having selenocysteine as its constituting amino acid in the cell. This further suggests the possibility of isolation and identification of unknown selenoproteins via DNAs hybridizing to SECIS of this invention. In the context of the present invention, analysis of mRNA can be performed by, for example, conventional technique such as Northern blotting assay, for example. In addition, nucleotide sequences can be amplified using DNAs of this invention as primers with mRNA of SECIS or genomic DNA as the template.

The design of nucleotide sequences to be used as probes and primers based on a given nucleotide sequence is routinely carried out by those skilled in the art. In the context of the present invention, the DNAs to be used for these purposes should have a chain length of at least 15 nucleotides, so as to be able to hybridize to the target sequence under stringent conditions . Preferably, they are oligonucleotides comprising 15 to 200 nucleotides, more preferably 25 to 100 nucleotides. Nucleotide sequences that achieve the specific hybridization need not be completely complementary to the targeted nucleotide sequence. Variations in the sequence are acceptableinasmuch as these variants exhibit the required specificity under stringent conditions. Oligonucleotides having the predetermined nucleotide sequences can be obtained by chemical synthesis, for example. These oligonucleotides can be tagged with labeling components, including, but not limited to, enzymes, fluorescent substances, luminous substances and radioisotopes as the occasion demands. For attachment (immobilization) and such of the DNAs to a solid phase to separate them from these labeling substances or labeling probes that did not hybridize, direct chemical binding methods are known. Alternatively, they can be indirectly bound to each other by.labeling oligonucleotides with ligands such as haptens or biotin, for example, and using antibodies recognizing these ligands and receptors for biotin, and so on.

Furthermore, the present invention also provides DNAs that are anti-sense to the DNA set forth in SEQ ID NO: 1, or portions thereof. The SECIS according to this invention bears an important role in selenoprotein expression, and its activity is supported by a stem-loop structure constructed in the mRNA (Fig. 4). Therefore, antisense sequences interfering with this structure will exert inhibitory action on the translation system of selenoprotein located upstream thereof. Since SECIS of this invention is derived from mRNAs of TxRIIs, antisense sequences according to this invention will function as expression inhibitors specific to TxRIIs.

In addition, the present invention also provides a method of screening for substances that regulate the expression of selenoproteins. Namely, host cells transformed with the selenoprotein expression vector according to the present invention enable the screening of substances regulating expression of the proteins. On bringing these cells into contact with candidate expression regulating compounds, the TxR activity in the cells or culture supernatant thereof is increased or decreased depending on the expression regulating activity of the candidate compounds. Therefore, it is possible to screen regulating substances that inhibit or stimulate the expression by comparing changes in this activity with that of untreated controls. Mammalian cell lines such as 293T are preferable as host cells.

Brief Description of the Drawings

Fig. 1 is a graphical representation of TxR activity, measured by the DTNB assay with recombinant TxRI or TxRIIα. Ordinate represents the absorbance at 412 nm, and abscissa the reaction time. (a) through (g) indicate expression products of the following vectors :

(a) pCMV6myc,
(b) pCMV6myc-TxRIIα,
(c) pCMV6myc-TxRIIαΔ3'UTR,
(d) pCMV6myc-TxRIIα3'UTR A.S.,
(e) pCMV6myc-TxRIIαCysΔ3'UTR sense,
(f) pCMV6myc-TxRI-flag, and
(g) pCMV6myc-TxRI-flag-3'UTR.

Fig. 2 is a graphical representation of TxR activity, measured by the insulin assay using recombinant TxRI or TxRIIα. Ordinate represents relative activity (%) to that of TxRIIα taken as 100, and abscissa the types of vectors. (a) through (g) indicate expression products of the following vectors:

(a) pCMV6myc,
(b) pCMV6myc-TxRIIα,
(c) pCMV6myc-TxRIIαΔ3'UTR,
(d) pCMV6myc-TxRIIα3'UTR A.S.,
(e) pCMV6myc-TxRIIαCysΔ3'UTR sense,
(f) pCMV6myc-TxRI-flag, and
(g) pCMV6myc-TxRI-flag-3'UTR.

Fig. 3 is a series of schematic diagrams showing the structure each of vectors (b) through (g) expressed in Examples, whose 3'UTR is artificially modified.

Fig. 4 is a schematic diagram showing the stem-loop structure formed by the SECIS according to this invention in the mRNA transcribed from the nucleotide sequence set forth in SEQ ID NO: 1.

Best Mode for Carrying out the Invention

The present invention will be described below in more detail with reference to examples.

To demonstrate that the active center of TxRIIs is the selenocysteine residue (SeCys) at the carboxyl terminal side, and that the 3'UTR thereof is essential for recognizing the UGA codon as the codon for SeCys, the following four types of TxRIIα expression vectors have been constructed. In addition, to prove that the 3'UTR of TxRIIα is also useful for other selenoproteins, a TxRI expression vector incorporated with the 3'UTR of TxRIIα in place of the 3'UTR of the already reported human TxRI has been constructed. Structures of mRNAs formed by transcription of these vectors are shown in Fig. 3.

Techniques routinely used in this invention have been all performed according to those described in J. Sambrook, E. F. Fritsch & T. Maniatis (1989) Molecular Cloning, a laboratory manual, second edition, Cold Spring Harbor Laboratory Press.

Cloning of Human TxRIIα cDNA

1. Construction of pCMV6myc-TxRIIα

The full-length gene containing the 3'UTR of TxRIIα (hereinafter referred to as TxRIIα) was subcloned into mammalian cell expression vector pCMV6myc. The pACT2-TxRIIα plasmid DNA isolated from the Human Placenta MATCHMAKER cDNA Library by the two-hybrid method was treated with the restriction enzymes EcoRI and XhoI, and a DNA fragment thus obtained was subcloned into the EcoRI/XhoI site of pCMV6myc.

The pCMV6myc vector has pcDNA3 (Invitrogen) as its basic skeleton, and is incorporated with a DNA encoding six repeated amino acid sequences, designated as the Myc tagged epitope, at HindIII and BamHI sites of its multi-cloning site (MCS). Genes incorporated into the MCS of this vector are transcribed or translated as a gene product with the Myc tag added to its N-terminal side. Therefore, the product can be detected by Western blot analysis, and such, or purified by immunoprecipitation, using antibody specific to the Myc tag.

2. Construction of pCMV6myc-TxRIIαΔ3'UTR

The gene sequence of TxRIIα deprived of the 3'UTR was subcloned into a mammalian cell expression vector pCMV6myc.

1) Designing primers:

The gene sequence of TxRIIα deprived of its 3'UTR was obtained by PCR method using the following primers, with the pACT2-TxRIIα plasmid DNA as the template. For the subsequent experiments, a 3'-primer was designed so as to yield cDNA encoding protein deprived of the final stop codon in its C-terminus amino acid sequence T-V-T-G-C-SeCys-G-stop.

5'-primer (pACT2 primer 4 (27mer), SEQ ID NO: 6);
5'-TAC CCA TAC GAT GTT CCA GAT TAC GCT-3', which is located upstream of MCS in the pACT2 vector.
3'-primer (TxRIIα-R1 (30mer), SEQ ID NO: 7);
5'-ATA CTC GAG CCC TCA GCA GCC TGT CAC CGT-3', wherein the *5'-terminal three nucleotides (ATA) are for conducting treatment with restriction enzyme smoothly, and the 5'-terminal nucleotides at 4th to 9th positions (CTCGAG) are the restriction enzyme XhoI site.

2) PCR conditions:

PCR was performed using a GeneAmp PCR System 2400 (PERKINELMER) according to the following program:

a) 94°C 5 min,
b) 94°C 1 min, 57°C 1 min and 72°C 2 min, 20 cycles, and
c) 72°C 10 min.

3) Sequence confirmation by cloning and sequencing:

PCR products were treated with the restriction enzymes BamHI and XhoI, and, after fractionation by agarose electrophoresis, were purified according to a standard method. The pCMV6myc vector was digested with the restriction enzymes BamHI and XhoI, fractionated by agarose electrophoresis, and purified by a usual method. Afterligation of these digests, *E. coli* strain DH5α was transformed with the resulting recombinant vector. Plasmid DNAs were collected using the alkaline-SDS method from the obtained colonies. Plasmid DNAs thus collected were cleaved with appropriate restriction enzymes to confirm the incorporation of desired PCR product into the vector by agarose electrophoresis. Recovered DNA was purified by the polyethylene glycol precipitation method to identify the PCR product inserted into the vector using a fluorescence sequencer (PERKINELMER) based on Sanger method. As a result, plasmid DNApCMV6myc-TxRIIαΔ3'UTR, which is a pCMV6myc vector inserted with the TxRIIα gene sequence deprived of the 3'UTR, was obtained.

3. Construction of pCMV6myc-TxRIIαCysΔ3'UTR

The gene sequence comprising TxRIIα deprived of its 3'UTR, in which the codon for SeCys, its putative active center, was substituted with that for cysteine (Cys), was subcloned into mammalian cell expression vector pCMV6myc. PCR was carried out with the pACT2-TxRIIα plasmid DNA as the template, using the following primers, to obtain the desired PCR product. For the subsequent experiments, the 3'-primer was designed to yield cDNA encoding an amino acid sequence having the C-terminus amino acid sequence T-V-T-G-C-SeCys-G-stop deprived of its final stop codon and comprising T-V-T-G-C-Cys-G.

5'-primer (pACT2 primer 4 (27mer), SEQ ID NO: 8);
5'-TAC CCA TAC GAT GTT CCA GAT TAC GCT-3', which is located upstream of MCS in the pACT2 vector.
3'-primer (TxRIIαCys-R1 (30mer), SEQ ID NO: 9);
5'-ATA CTC GAG CCC ACA GCA GCC TGT CAC CGT-3', wherein the *5'-terminal three nucleotides (ATA) are for conducting treatment with restriction enzyme smoothly, and the 5'-terminal nucleotides at 4th to 9th positions (CTCGAG) are the restriction enzyme XhoI site. 2) PCR conditions:

PCR was performed using a GeneAmp PCR System 2400 (PERKINELMER) according to the following program:

a) 94°C 5 min,
b) 94°C 1 min, 57°C 1 min and 72°C 2 min, 20 cycles, and
c) 72°C 10 min.

3) Sequence confirmation by cloning and sequencing:

PCR products were treated with the restriction enzymes BamHI and XhoI, fractionated by agarose electrophoresis, and purified according to a standard method. The pCMV6myc vector was digested with the restriction enzymes BamHI and XhoI, fractionated by agarose electrophoresis, and purified by a usual method. After ligating these digests, *E. coli* strain DH5α was transformed with the resulting recombinant vector. The plasmid DNA was purified by a standard method from the obtained colonies, and the PCR product in the vector was identified using a fluorescence sequencer (PERKINELMER), based on the Sanger method. As a result, the plasmid DNA, pCMV6myc-TxRIIαCysΔ3'UTR, which is a pCMV6myc vector incorporated with the TxRIIα gene sequence deprived of the 3'UTR and having the codon for SeCys replaced at the putative active center for the codon for cysteine (Cys), was obtained.

Cloning of the 3'UTR in the human TxRIIα

The following primers were synthesized from the DNA sequence set forth in SEQ ID NO: 2 (Japanese Patent Application No. Hei 10-310422), and the 3'UTR sequence of TxRIIα was obtained by PCR. The primers used were those that can amplify the 358 nucleotides at the 1573rd to the 1930th positions in the sequence set forth in SEQ ID NO: 2.

1) Preparation of primers:

5'-primer (TxRIIα3'UTR-F1 (30mer), SEQ ID NO: 10)
5'-ATA TCT AGA TAA GCG CCA TCC CTG CAG GCC-3', wherein the *5'-terminal three nucleotides (ATA) are for conducting treatment with restriction enzymes smoothly, and the 5'-terminal nucleotides at 4th to 9th positions (TCTAGA) are the restriction enzyme XbaI site.
3'-primer (TxRIIα3'UTR-R1 (30mer), SEQ ID NO: 11);
5'-GCG TCT AGA CAC ACT TCA GAA AAA GTA CCC-3', wherein the *5'-terminal three nucleotides (GCG) are for conducting treatment with restriction enzymes smoothly, and the 5'-terminal nucleotides at 4th to 9th positions (TCTAGA) are the restriction enzyme XbaI site.

2) PCR:

Using the plasmid pACT2-TxRIIα, inserted with a full-length cDNA containing the 3'UTR of TxRIIα, as the template DNA, the 3'UTR was amplified by PCR. PCR was carried out using a GeneAmp PCR System 2400 (PERKINELMER) according to the following program:

a) 94°C 5 min,
b) 94°C 1 min, 58°C 3 min and 72°C 1 min, 1 cycle,
c) 94°C 1 min, 66°C 0.5 min and 72°C 0.5 min, 20 cycles, and
d) 72°C 10 min.

3) Cloning of PCR product into pSL-1180 vector:

After PCR, the amplified DNA fragment was identified by electrophoresis in 1% agarose, and treated with the restriction enzyme XbaI. After the DNA fragment treated with the restriction enzyme was electrophoresed in 1% agarose, it was excised from the gel, and purified by the glass matrix method (BIO101, GeneClean). pSL-1180 (Amersham Pharmacia Biotech) was treated with the restriction enzyme XbaI, and then purified by the alkaline phosphatase treatment. The XbaI-treated vector and DNA fragment were ligated by a usual method. The *E. coli* strain DH5α was transformed with the resulting plasmid according to a standard method, and the plasmid DNA was purified from colonies thus obtained using the polyethylene glycol precipitation method. The PCR product in the vector was identified by a fluorescence sequencer (PERKINELMER), based on Sanger method. Thus, a plasmid DNA, pSL-3'UTR, namely, the pSL-1180 vector incorporated with the 3'UTR sequence of.human TxRIIα gene, was obtained.

4. Construction of pCMV6myc-TxRIIα3'UTR A.S.

pSL-3'UTR was treated with the restriction enzyme XbaI to excise the 3'UTR of TxRIIα.

pCMV6myc-TxRIIαΔ3'UTR was digested with the restriction enzyme XbaI followed by the treatment with alkaline phosphatase. These two digests were ligated according to a standard method, and *E. coli* strain DH5α was transformed with the resulting plasmid. The plasmid DNA was purified from colonies thus obtained using the polyethylene glycol precipitation method, and the PCR product within the vector was identified by a fluorescence sequencer (PERKINELMER) , based on Sanger method. As a result, the pCMV6myc-TxRIIα3'UTR A.S., wherein the 3'UTR of TxRIIα is incorporated in the reverse (antisense) direction downstream of TxRIIαΔ3'UTR, was obtained.

5. Construction of pCMV6myc-TxRIIαCys3'UTRsense and A.S.

pSL-3'UTR was digested with the restriction enzyme XbaI to excise the 3'UTR of TxRIIα.

pCMV6myc-TxRIIαCysΔ3'UTR was digested with the restriction enzyme XbaI followed by the treatment with alkaline phosphatase. These two digests were ligated according to a usual method, and the *E. coli* strain DH5α was transformed with the resulting plasmid. The plasmid DNA was purified from colonies thus obtained using the polyethylene glycol precipitation method, and the PCR product within the vector was identified by a fluorescence sequencer (PERKINELMER), based on Sanger method. As a result, a pCMV6myc-TxRIIαCys3'UTRsense, wherein the 3'UTR of TxRIIα is incorporated in the normal (sense) direction downstream of TxRIIαCysΔ3'UTR, was obtained.

Cloning of human thioredoxin reductase I

1. Construction of pCMV6myc-TxRI-flag

1) Preparation of primers:

PCR primers described below were synthesized from the previously reported DNA sequence (GenBank accession number D88687) of human thioredoxin reductase I (hereinafter abbreviated as TxRI). For the subsequent experiments, a 3'-primer was designed to yield cDNA encoding an amino acid sequence comprising the C-terminal amino acid sequence, L-Q-A-G-C-SeCys-G-stop, from which the final stop codon is removed.

5'-primer (TxRI-F1 (30mer), SEQ ID NO: 12)
5'-ATA GGA TCC ATG TCA TGT GAG GAC GGT CGG-3', wherein the *5'-terminal three nucleotides (ATA) are for conducting treatment with restriction enzymes smoothly, and the 5'-terminal nucleotides at 4th to 9th positions (GGATCC) are the restriction enzyme BamHI site.

3'-primer (TxRI-R1 (30mer), SEQ ID NO: 13);
5'-ATA CTC GAG ACC TCA GCA GCC AGC CTG GAG-3', wherein the *5'-terminal three nucleotides (ATA) are for conducting treatment with restriction enzymes smoothly, and the 5'-terminal nucleotides at 4th to 9th positions (CTCGAG) are the restriction enzyme XhoI site.

2) PCR:

A full length human TxRI gene was amplified by PCR using a plasmid DNA purified from the Human Placenta MATCHMAKER cDNA library purchased from CLONTECH as the template DNA. PCR was performed using a Gene-Amp PCR System 2400 (PERKINELMER) according to the following program:

a) 94°C 5 min,
b) 94°C 1 min, 56°C 3 min and 72°C 2 min, 1 cycle,
c) 94°C 1 min, 65°C 1 min and 72°C 2 min, 35 cycles, and
d) 72°C 10 min.

3) Cloning of PCR product into pCMV6myc-flag vector:

3)-1 Purification of PCR product

After PCR, the amplified DNA fragment was identified by electrophoresis in 1% agarose, and then digested with the restriction enzymes BamHI and XhoI. The restriction enzyme-treated DNA fragment was electrophoresed in 1% agarose, excised from the gel, and purified by the glass matrix method (BI0101, GeneClean).

3)-2 pCMV6myc-flag vector

The pCMV6myc-flag vector has the pcDNA3 (Invitrogen) as the basic skeleton, which is incorporated with a DNA encoding six repeated amino acid sequences, referred to as the Myc tagged epitope, at the HindIII and BamHI sites of its multicloning site (MCS), and also with another DNA encoding an amino acid sequence, designated as the flag epitope, at the XhoI and XbaI sites downstream of the Myc tag gene. Therefore, a gene incorporated between the Myc tag and flag tag genes would express a gene product ligated to the Myc tag at its N-terminus and the flag tag at its C-terminus.
Since products of genes incorporated into MCS can be detected with antibodies to these Myc and flag tags even when no antibody to the product is available, these tags are widely used in a variety of expression systems.

3)-3 Ligation and transformation of *E. coli*

The PCR product which had been cleaved with the restriction enzymes BamHI and XhoI and then purified were ligated to the pCMV6myc-flag vector, which had been cleaved with the same restriction enzymes and purified according to a usual method. The *E. coli* strain DH5α was transformed with the above plasmid according to the standard method (Hanahan, D. (1983) Studies on transformation of Escherichia coli with plasmids, J. Mol. Biol. 166: 557), and the plasmid DNA was recovered from colonies thus obtained using the alkaline SDS method. Plasmid DNA thus collected was cleaved with appropriate restriction enzymes, and the incorporation of desired PCR product into the vector was confirmed by agarose electrophoresis. Recovered DNA was purified by the polyethylene glycol precipitation method, and PCR product in the vector was identified using a fluorescence sequencer (PERKINELMER) based on Sanger method. Thus, a plasmid DNA, pCMV6myc-TxRI-flag, that is, the pCMV6myc-flag vector incorporated with a full length human TxRI gene, was obtained.

2. Construction of pCMV6myc-TxRI-flag-3'UTR

pSL-3'UTR was treated with the restriction enzyme XbaI to excise the 3'UTR of TxRIIα.
pCMV6myc-TxRI-flag was treated with the restriction enzyme XbaI, followed by the treatment with alkaline phosphatase. These digests were ligated by a standard method. *E. coli* strain DH5α was transformed with the above plasmid according to a standard method. The plasmid DNA was recovered from colonies thus obtained and purified by the polyethylene glycol precipitation method. The PCR product within the vector was identified using a fluorescence sequencer (PERKINELMER) based on Sanger method. Thus, the pCMV6myc-TxRI-flag-3'UTR, in which the 3'UTR of TxRIIα was incorporated downstream of TxRI gene in the normal (sense) direction was obtained.

Purification and activity assay of recombinant TxRI and TxRIIα proteins

1) Purification of Myc-tagged fusion proteins between TxRI and TxRIIα:

(a) pCMV6myc,
(b) pCMV6myc-TxRIIα,
(c) pCMV6myc-TxRIIαΔ3'UTR,
(d) pCMV6myc-TxRIIα3'UTR A.S.,
(e) pCMV6myc-TxRIIαCysΔ3'UTR sense,
(f) pCMV6myc-TxRI-flag, and
(g) pCMV6myc-TxRI-flag-3'UTR.

Cultured cell 293T, derived from the human fetal kidney cells, were transformed with the mammalian cell expression plasmids (a) to (g) by the standard lipofection method. Cells were recovered 48 h after the transformation to prepare cell extracts, to which the anti-Myc monoclonal antibody bound to protein A sepharose was added, and the resulting mixture was gently stirred at 4°C for 2 h. The Myc-tagged fusion protein bound to the anti-Myc monoclonal antibody immobilized to protein A sepharose was precipitated by centrifugation, and, after the removal of supernatant, washed several times with NETN buffer (10 mM Tris-HCl, 1 mM EDTA, 0,5% NP-40 and 150 mM NaCl). The supernatant was completely removed, and diluted in 100 mM potassium phosphate, pH 7.0, containing 10 mM EDTA and 0.2 mg/ml bovine serum albumin.

2) Activity assay

Activity assay of thioredoxin reductase was performed by the following two methods according to the standard technique (Holmgren, A. et al. Methods Enzymol. 252: 199, 1995).

Method 1. 5,5'-Dithiobis(2-nitrobenzoic acid) (DTNB) assay

In the DTNB assay, the TxR activity to produce TNB from DTNB is measured by the thiol absorbance at 412 nm based on the following formula:

$$DTNB + NADPH + H^{+} \rightarrow 2TNB + NADP^{+}$$

Assay buffer 1:

100 mM potassium phosphate, pH 7.0, containing 10 mM EDTA, 0.25 mM NADPH, 0.2 mg/ml bovine serum albumin (BSA), 1% ethanol, and 1 mM DTNB.

Purified Myc-tagged fusion protein (1 to 50 µl) was added to the assay buffer 1 to make a total volume 1.0 ml. The absorbance at 412 nm of the assay mixture was followed at 25°C for 20 min. Results are shown in Fig. 1.

Method 2. Insulin assay

Assay buffer 2:

50 mM phosphate buffer pH 7.0 containing 20 mM EDTA, 80 mM insulin, 0.25 mM NADPH, 16 mM *E. coli* thioredoxin-S2.

Purified Myc-tagged fusion protein (1 to 50 µl) was added to the assay buffer 2 to make a total volume 1.0 ml. The oxidation of NADPH was measured at 30°C for 5 min as the absorbance at 340 nm. Trx is reduced by the TxR activity, and the reduced Trx further promotes the reduction of insulin. In this case, TxR activity can be measured from the amount of NADPH to be oxidized. The amount of NADPH which was oxidized was calculated from the following equation. Relative values of expression products due to each of vectors were expressed as percentage of the TxRIIα activity taken as 100%, and were graphically represented (Fig. 2).

$$\Delta A340 \times 0.5 / 6.2$$

Results

No activity was observed when the 3'UTR of TxRIIα was omitted (c), and also when the 3'UTR of TxRIIα was inserted in the antisense direction (d). No activity was also observed when SeCys was substituted with Cys (e) regardless of the absence or presence of its 3'UTR. Likewise, in the case where the 3'UTR was missing from TxRI (f) , no activity was detected. However, when the 3'UTR of TxRIIα was added to TxRI, the activity of TxRI was observed (g)
As these experiments demonstrate, the fact that the enzyme activity was lost, when the SeCys residue at the C-terminal side of TxRIIα was substituted with Cys, strongly suggests that the active center of TxRIIα is the SeCys residue. By the loss of enzyme activity due to deletion of its 3'UTR, it could be confirmed that the 3'UTR is essential for recognizing the UGA codon as that for SeCys. Furthermore, the fact that TxR activity was gained by adding the 3'UTR of TxRIIα to other selenoprotein TxRI demonstrates that the 3'UTR of TxRIIα is useful for incorporating the SeCys residue into other selenoproteins.

Industrial Applicability

The SECIS of this invention allows for the translation of selenoproteins without fail while having a sequence of less than 360 b. This size (360b) is extremely small as compared with known SECISs, comprising, for example, several thousands nucleotides. Nucleotide sequences of the size of the present invention will not exert unfavorable effects under usual conditions when inserted into vectors. Furthermore, such a SECIS itself is easily synthesized.
SECIS of this invention comprise a sequence having a broad activity universally acting on not only TxRIIs from which it was derived but also overall selenoproteins.

## Claims

1. A DNA comprising the nucleotide sequence as defined in SEQ ID NO: 1.

2. A DNA comprising the nucleotide sequence as defined in SEQ ID NO: 1, wherein one or more nucleotides are substituted, deleted, added, and/or inserted, that inserts selenocysteine for the TGA codon contained in the translational region located upstream of said DNA.

3. A DNA that hybridizes under stringent conditions to a DNA having the nucleotide sequence as defined in SEQ ID NO: 1, and that inserts selenocysteine for the TGA codon contained in the translational region located upstream of said DNA.

4. A DNA that hybridizes to the nucleotide sequence as defined in SEQ ID NO: 1 and that has a chain length of at least 15 nucleotides.

5. A selenoprotein expression vector comprising the following elements:

   a) a cloning site,to which a DNA encoding a selenoprotein amino acid sequence can be inserted,
   b) the DNA of any one of claims 1, 2 and 3 ligated downstream of said cloning site, and
   c) a regulatory region required for protein expression.

6. The selenoprotein expression vector of claim 5, further comprising a DNA encoding a selenoprotein amino acid sequence inserted at the cloning site.

7. A transformant transformed with the selenoprotein expression vector of claim 6.

8. A method for preparing a selenoprotein comprising the steps of culturing a transformant of claim 7 and collecting the selenoprotein.

9. A method for preparing a selenoprotein of claim 8, wherein the selenoprotein is an enzyme containing a selenocysteine in its active center.

10. The method for preparing a selenoprotein of claim 9, wherein said enzyme containing selenocysteine in its active center is thioredoxin reductase.

11. A DNA that is antisense to the DNA of claim 1 or a portion thereof.

**12.** A method of screening for substances that regulate the expression of selenoproteins, comprising the steps of:

a) bringing candidate compounds into contact with the transformant of claim 7,
b) measuring the selenoprotein contained in the host cells or the culture supernatant thereof and comparing it with controls, and
c) selecting a compound that alters the expression level of selenoproteins by comparison with controls.

Figure 1

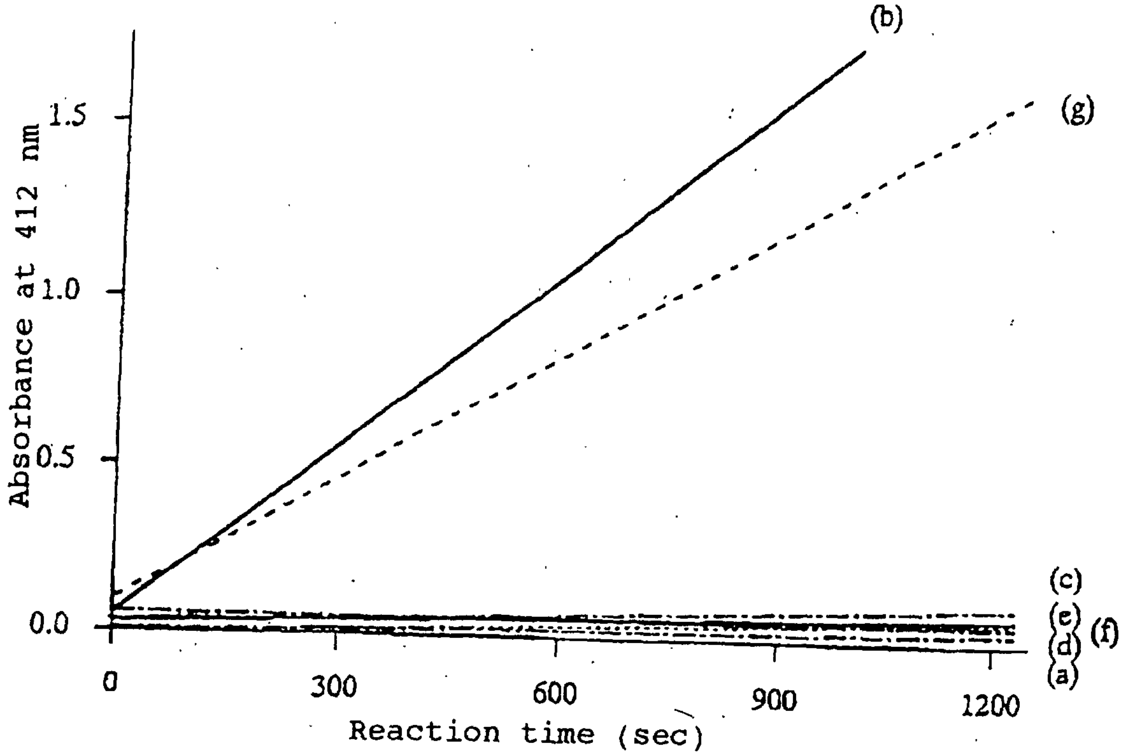

Absorbance at 412 nm
1.5
1.0
0.5
0.0
0
300
600
900
1200
Reaction time (sec)
(b)
(g)
(c)
(e)
(d)
(a)
(f)

Figure 2

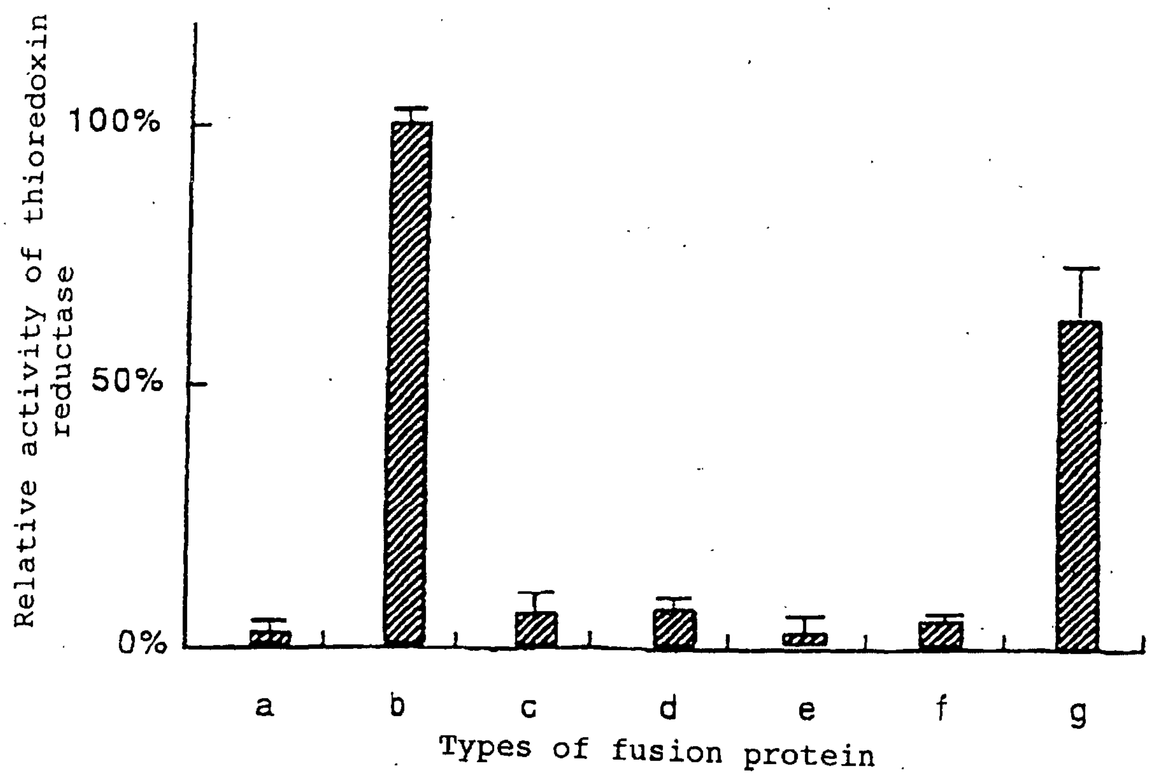
Relative activity of thioredoxin reductase
100%
50%
0%
a
b
c
d
e
f
g
Types of fusion protein

Figure 3

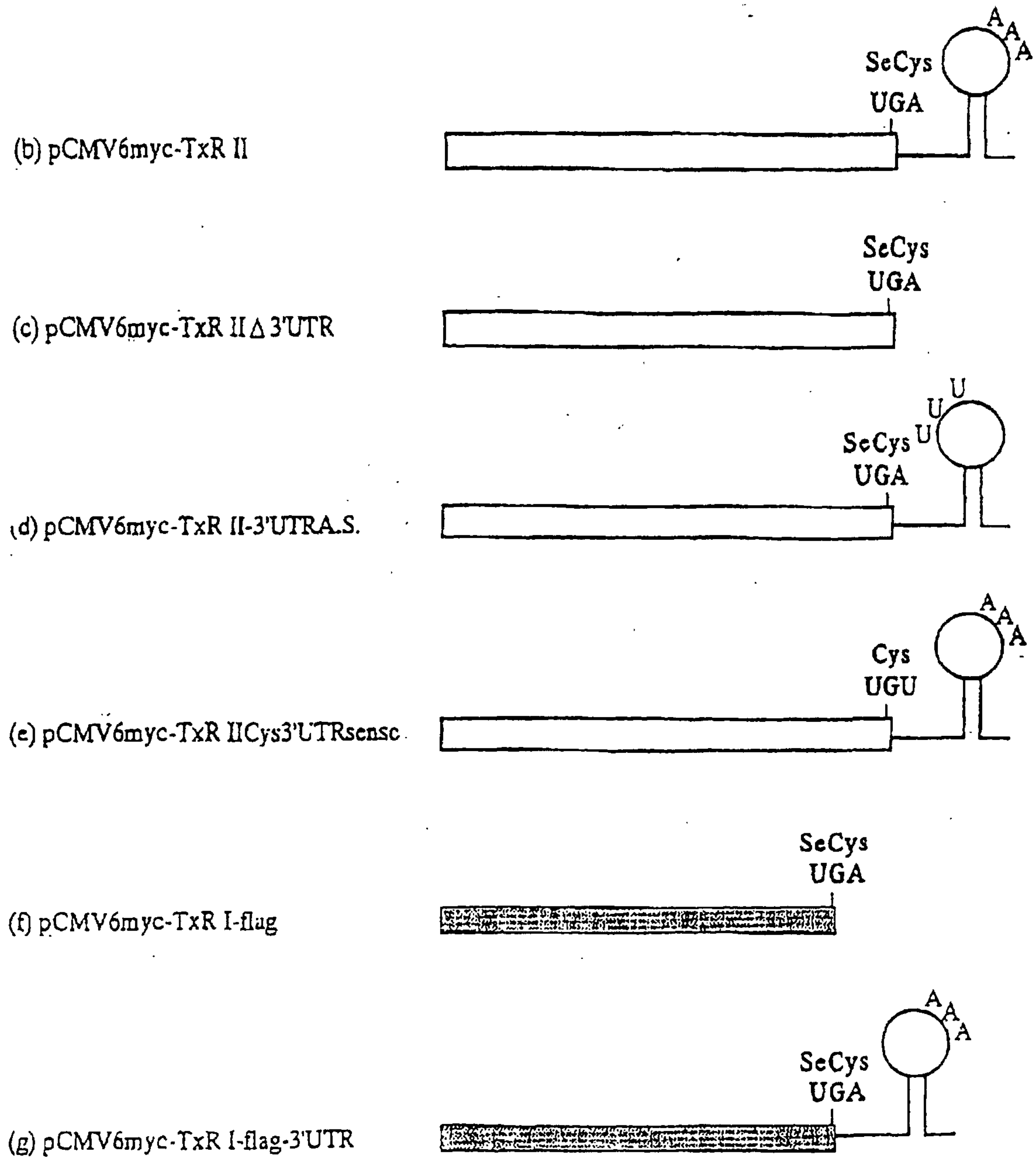
(b) pCMV6myc-TxR II
SeCys
UGA
AAA
(c) pCMV6myc-TxR II Δ 3'UTR
SeCys
UGA
(d) pCMV6myc-TxR II-3'UTRA.S.
SeCys
UGA
UUU
(e) pCMV6myc-TxR IICys3'UTRsense
Cys
UGU
AAA
(f) pCMV6myc-TxR I-flag
SeCys
UGA
(g) pCMV6myc-TxR I-flag-3'UTR
SeCys
UGA
AAA

Figure 4

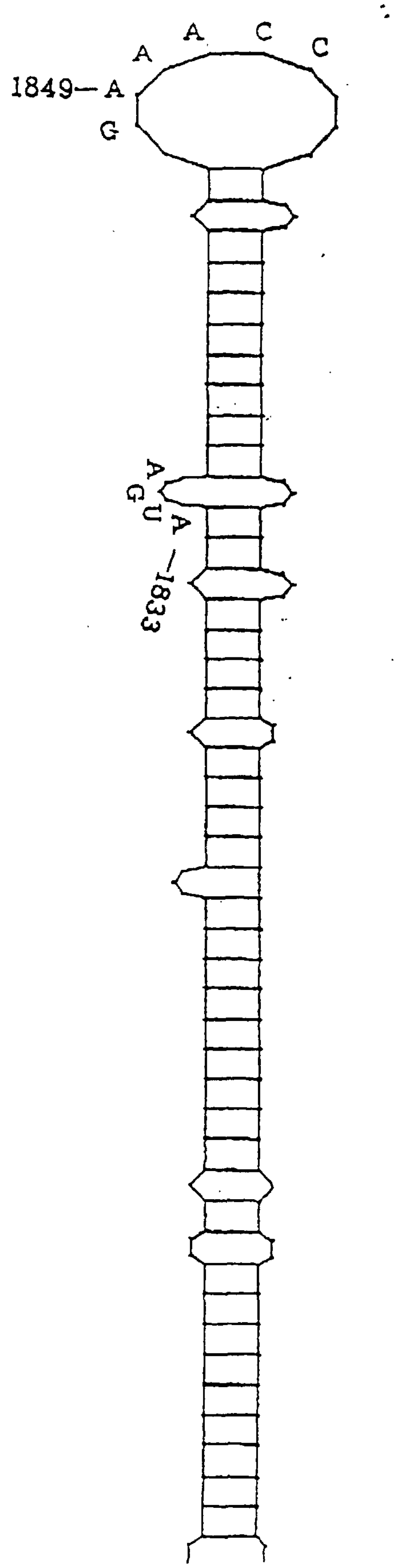

## INTERNATIONAL SEARCH REPORT

International application No. PCT/JP99/05984

A. CLASSIFICATION OF SUBJECT MATTER
Int.Cl[7] C12N15/11, C12N15/63, C12N5/10, C12P21/02, C12N9/02

According to International Patent Classification (IPC) or to both national classification and IPC

B. FIELDS SEARCHED

Minimum documentation searched (classification system followed by classification symbols)
Int.Cl[7] C12N15/11, C12N15/63, C12N5/10, C12P21/02, C12N9/02

Documentation searched other than minimum documentation to the extent that such documents are included in the fields searched

Electronic data base consulted during the international search (name of data base and, where practicable, search terms used)
REGISTRY/CA (STN)
DDBJ/GenBank/EMBL/GeneSeq
Swissprot/PIR/GeneSeq

C. DOCUMENTS CONSIDERED TO BE RELEVANT

| Category* | Citation of document, with indication, where appropriate, of the relevant passages | Relevant to claim No. |
|---|---|---|
| A | Annu. Rev. Biochemistry Vol. 65 (1996) Stadtman T. C. "Selenocysteine", p. 83-100 | 1-12 |
| A | FEBS Letters Vol. 373 (1995) Gasdaska P. Y. et al., "Cloning and sequencing of a human thioredoxin reductase", p. 5-9 | 1-12 |
| A | J. Biol. Chem. Vol. 272 No.4 (1997) Koishi R. et al., "cloning and Characterization of a Novel Oxidoreductase KDRF from a Human Bone Marrow-derived Stromal Cell Line KM-102", p. 2570-2577 | 1-12 |
| P,X<br>P,A | J. Biol. Chem. Vol. 274 No. 35 (Aug.1999) Sun Q. A. et al., "Redox regulation of cell signaling by selenocysteine in mammalian thioredoxin reductases", p. 24522-24530 | 1-4,11<br>5-10,12 |
| P,X<br>P,A | FEBS Letters Vol. 442 No. 1 (Jan.1999) Gasdaska P. Y. et al., "Cloning, sequencing and functional expression of a novel human thioredoxin reductase", p. 105-111 | 1-4,11<br>5-10,12 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>27 December, 1999 (27.12.99) | Date of mailing of the international search report<br>11 January, 2000 (11.01.00) |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP99/05984

C (Continuation). DOCUMENTS CONSIDERED TO BE RELEVANT

| Category* | Citation of document, with indication, where appropriate, of the relevant passages | Relevant to claim No. |
|---|---|---|
| P,X<br>P,A | Eur. J. Biochem. Vol. 261 No. 2 (Apr.1999) Miranda-Vizuete A. et al., "Human mitochondrial thioredoxin reductase cDNA cloning, expression and genomic organization", p. 405-412 | 1-4,11<br>5-10,12 |

Form PCT/ISA/210 (continuation of second sheet) (July 1992)

SEQUENCE LISTING

<110> Medical & Biological Laboratories Co., Ltd.

<120> Selenocystein Insertion Sequence

<130> M3-010PCT

<140>

<141>

<150> JP 1998-310422

<151> 1998-10-30

<150> JP 1998-325344

<151> 1998-11-16

<160> 13

<170> PatentIn Ver. 2.0

<210> 1

<211> 358

<212> DNA

<213> Homo sapiens

<400> 1

taagcgccat ccctgcaggc cagggcacac ggtgcgcccg ccgccagctc ctcggaggcc 60

agacccagga tggctgcagg ccaggtttgg ggggcctcaa ccctctcctg gagcgcctgt 120

gagatggtca gcgtggagcg caagtgctgg acgggtggcc cgtgtgcccc acagggatgg 180

ctcaggggac tgtccacctc acccctgcac ctttcagcct ttgccgccgg gcaccccccc 240

caggctcctg gtgccggatg atgacgacct gggtggaaac ctaccctgtg ggcacccatg 300

tccgagcccc ctggcatttc tgcaatgcaa ataaagaggg tactttttct gaagtgtg 358

<210> 2

<211> 1959

<212> DNA

<213> Homo sapiens

<220>

<221> CDS

<222> (10)..(1572)

<220>

<221> 3'UTR

<222> (1573)..(1930)

<220>

<221> misc_structure

<222> (1567)..(1569)

<223> tga is translated to selenosystein, shown by Xaa.

<400> 2

atggccgca atg gcg gtg gcg ctg cgg gga tta gga ggg cgc ttc cgg tgg 51
Met Ala Val Ala Leu Arg Gly Leu Gly Gly Arg Phe Arg Trp
1 5 10

cgg acg cag gcc gtg gcg ggc ggg gtg cgg ggc gcg gcg cgg ggc gca 99
Arg Thr Gln Ala Val Ala Gly Gly Val Arg Gly Ala Ala Arg Gly Ala
15 20 25 30

gca gca ggt cag cgg gac tat gat ctc ctg gtg gtc ggc ggg gga tct 147
Ala Ala Gly Gln Arg Asp Tyr Asp Leu Leu Val Val Gly Gly Gly Ser
35 40 45

ggt ggc ctg gct tgt gcc aag gag gcc gcc cag ctg gga agg aag gtg 195
Gly Gly Leu Ala Cys Ala Lys Glu Ala Ala Gln Leu Gly Arg Lys Val
50 55 60

gcc gtg gtg gac tac gtg gaa cct tct ccc caa ggc acc cgg tgg ggc 243
Ala Val Val Asp Tyr Val Glu Pro Ser Pro Gln Gly Thr Arg Trp Gly
65 70 75

ctc ggc ggc acc tgc gtc aac gtg ggc tgc atc ccc aag aag ctg atg 291
Leu Gly Gly Thr Cys Val Asn Val Gly Cys Ile Pro Lys Lys Leu Met
80 85 90

cac cag gcg gca ctg ctg gga ggc ctg atc caa gat gcc ccc aac tat 339
His Gln Ala Ala Leu Leu Gly Gly Leu Ile Gln Asp Ala Pro Asn Tyr
95 100 105 110

ggc tgg gag gtg gcc cag ccc gtg ccg cat gac tgg agg aag atg gca 387
Gly Trp Glu Val Ala Gln Pro Val Pro His Asp Trp Arg Lys Met Ala
115 120 125

gaa gct gtt caa aat cac gtg aaa tcc ttg aac tgg ggc cac cgt gtc 435
Glu Ala Val Gln Asn His Val Lys Ser Leu Asn Trp Gly His Arg Val
130 135 140

cag ctt cag gac aga aaa gtc aag tac ttt aac atc aaa gcc agc ttt 483
Gln Leu Gln Asp Arg Lys Val Lys Tyr Phe Asn Ile Lys Ala Ser Phe
145 150 155

gtt gac gag cac acg gtt tgc ggc gtt gcc aaa ggt ggg aaa gag att 531

Val Asp Glu His Thr Val Cys Gly Val Ala Lys Gly Gly Lys Glu Ile
160 165 170

ctg ctg tca gcc gat cac atc atc att gct act gga ggg cgg ccg aga 579
Leu Leu Ser Ala Asp His Ile Ile Ile Ala Thr Gly Gly Arg Pro Arg
175 180 185 190

tac ccc acg cac atc gaa ggt gcc ttg gaa tat gga atc aca agt gat 627
Tyr Pro Thr His Ile Glu Gly Ala Leu Glu Tyr Gly Ile Thr Ser Asp
195 200 205

gac atc ttc tgg ctg aag gaa tcc cct gga aaa acg ttg gtg gtc ggg 675
Asp Ile Phe Trp Leu Lys Glu Ser Pro Gly Lys Thr Leu Val Val Gly
210 215 220

gcc agc tat gtg gcc ctg gag tgt gct ggc ttc ctc acc ggg att ggg 723
Ala Ser Tyr Val Ala Leu Glu Cys Ala Gly Phe Leu Thr Gly Ile Gly
225 230 235

ctg gac acc acc atc atg atg cgc agc atc ccc ctc cgc ggc ttc gac 771
Leu Asp Thr Thr Ile Met Met Arg Ser Ile Pro Leu Arg Gly Phe Asp
240 245 250

cag caa atg tcc tcc atg gtc ata gag cac atg gca tct cat ggc acc 819
Gln Gln Met Ser Ser Met Val Ile Glu His Met Ala Ser His Gly Thr
255 260 265 270

cgg ttc ctg agg ggc tgt gcc ccc tcg cgg gtc agg agg ctc cct gat 867
Arg Phe Leu Arg Gly Cys Ala Pro Ser Arg Val Arg Arg Leu Pro Asp
275 280 285

ggc cag ctg cag gtc acc tgg gag gac agc acc acc ggc aag gag gac 915
Gly Gln Leu Gln Val Thr Trp Glu Asp Ser Thr Thr Gly Lys Glu Asp
290 295 300

acg ggc acc ttt gac acc gtc ctg tgg gcc ata ggt cga gtc cca gac 963
Thr Gly Thr Phe Asp Thr Val Leu Trp Ala Ile Gly Arg Val Pro Asp
305 310 315

acc aga agt ctg aat ttg gag aag gct ggg gta gat act agc ccc gac 1011
Thr Arg Ser Leu Asn Leu Glu Lys Ala Gly Val Asp Thr Ser Pro Asp
320 325 330

act cag aag atc ctg gtg gac tcc cgg gaa gcc acc tct gtg ccc cac 1059
Thr Gln Lys Ile Leu Val Asp Ser Arg Glu Ala Thr Ser Val Pro His
335 340 345 350

atc tac gcc att ggt gac gtg gtg gag ggg cgg cct gag ctg aca ccc 1107
Ile Tyr Ala Ile Gly Asp Val Val Glu Gly Arg Pro Glu Leu Thr Pro
355 360 365

aca gcg atc atg gcc ggg agg ctc ctg gtg cag cgg ctc ttc ggc ggg 1155

Thr Ala Ile Met Ala Gly Arg Leu Leu Val Gln Arg Leu Phe Gly Gly

370 375 380

tcc tca gat ctg atg gac tac gac aat gtt ccc acg acc gtc ttc acc 1203

Ser Ser Asp Leu Met Asp Tyr Asp Asn Val Pro Thr Thr Val Phe Thr

385 390 395

cca ctg gag tat ggc tgt gtg ggg ctg tcc gag gag gag gca gtg gct 1251

Pro Leu Glu Tyr Gly Cys Val Gly Leu Ser Glu Glu Glu Ala Val Ala

400 405 410

cgc cac ggg cag gag cat gtt gag gtc tat cac gcc cat tat aaa cca 1299

Arg His Gly Gln Glu His Val Glu Val Tyr His Ala His Tyr Lys Pro

415 420 425 430

ctg gag ttc acg gtg gct gga cga gat gca tcc cag tgt tat gta aag 1347

Leu Glu Phe Thr Val Ala Gly Arg Asp Ala Ser Gln Cys Tyr Val Lys

435 440 445

atg gtg tgc ctg agg gag ccc cca cag ctg gtg ctg ggc ctg cat ttc 1395

Met Val Cys Leu Arg Glu Pro Pro Gln Leu Val Leu Gly Leu His Phe

450 455 460

ctt ggc ccc aac gca ggc gaa gtt act caa gga ttt gct ctg ggg atc 1443

Leu Gly Pro Asn Ala Gly Glu Val Thr Gln Gly Phe Ala Leu Gly Ile

465 470 475

aag tgt ggg gct tcc tat gcg cag gtg atg cgg acc gtg ggt atc cat 1491
Lys Cys Gly Ala Ser Tyr Ala Gln Val Met Arg Thr Val Gly Ile His
480 485 490

ccc aca tgc tct gag gag gta gtc aag ctg cgc atc tcc aag cgc tca 1539
Pro Thr Cys Ser Glu Glu Val Val Lys Leu Arg Ile Ser Lys Arg Ser
495 500 505 510

ggc ctg gac ccc acg gtg aca ggc tgc tga ggg taagcgccat ccctgcaggc 1592
Gly Leu Asp Pro Thr Val Thr Gly Cys Xaa Gly
515 520

cagggcacac ggtgcgcccg ccgccagctc ctcggaggcc agacccagga tggctgcagg 1652

ccaggtttgg ggggcctcaa ccctctcctg gagcgcctgt gagatggtca gcgtggagcg 1712

caagtgctgg acgggtggcc cgtgtgcccc acagggatgg ctcaggggac tgtccacctc 1772

acccctgcac ctttcagcct ttgccgccgg gcaccccccc caggctcctg gtgccggatg 1832

atgacgacct gggtggaaac ctaccctgtg ggcacccatg tccgagcccc ctggcatttc 1892

tgcaatgcaa ataaagaggg tactttttct gaagtgtgta aaaaaaaaaa aaaaaaaaaa 1952

aaaaaaa 1959

<210> 3

<211> 519

<212> PRT

<213> Homo sapiens

<223> Xaa(520) means selenosysteine.

<400> 3

Met Ala Val Ala Leu Arg Gly Leu Gly Gly Arg Phe Arg Trp Arg Thr
1 5 10 15

Gln Ala Val Ala Gly Gly Val Arg Gly Ala Ala Arg Gly Ala Ala Ala
20 25 30

Gly Gln Arg Asp Tyr Asp Leu Leu Val Val Gly Gly Gly Ser Gly Gly
35 40 45

Leu Ala Cys Ala Lys Glu Ala Ala Gln Leu Gly Arg Lys Val Ala Val
50 55 60

Val Asp Tyr Val Glu Pro Ser Pro Gln Gly Thr Arg Trp Gly Leu Gly
65 70 75 80

Gly Thr Cys Val Asn Val Gly Cys Ile Pro Lys Lys Leu Met His Gln
85 90 95

Ala Ala Leu Leu Gly Gly Leu Ile Gln Asp Ala Pro Asn Tyr Gly Trp

100 105 110

Glu Val Ala Gln Pro Val Pro His Asp Trp Arg Lys Met Ala Glu Ala

115 120 125

Val Gln Asn His Val Lys Ser Leu Asn Trp Gly His Arg Val Gln Leu

130 135 140

Gln Asp Arg Lys Val Lys Tyr Phe Asn Ile Lys Ala Ser Phe Val Asp

145 150 155 160

Glu His Thr Val Cys Gly Val Ala Lys Gly Gly Lys Glu Ile Leu Leu

165 170 175

Ser Ala Asp His Ile Ile Ile Ala Thr Gly Gly Arg Pro Arg Tyr Pro

180 185 190

Thr His Ile Glu Gly Ala Leu Glu Tyr Gly Ile Thr Ser Asp Asp Ile

195 200 205

Phe Trp Leu Lys Glu Ser Pro Gly Lys Thr Leu Val Val Gly Ala Ser

210 215 220

Tyr Val Ala Leu Glu Cys Ala Gly Phe Leu Thr Gly Ile Gly Leu Asp

225 230 235 240

Thr Thr Ile Met Met Arg Ser Ile Pro Leu Arg Gly Phe Asp Gln Gln

245 250 255

Met Ser Ser Met Val Ile Glu His Met Ala Ser His Gly Thr Arg Phe

260 265 270

Leu Arg Gly Cys Ala Pro Ser Arg Val Arg Arg Leu Pro Asp Gly Gln

275 280 285

Leu Gln Val Thr Trp Glu Asp Ser Thr Thr Gly Lys Glu Asp Thr Gly

290 295 300

Thr Phe Asp Thr Val Leu Trp Ala Ile Gly Arg Val Pro Asp Thr Arg

305 310 315 320

Ser Leu Asn Leu Glu Lys Ala Gly Val Asp Thr Ser Pro Asp Thr Gln

325 330 335

Lys Ile Leu Val Asp Ser Arg Glu Ala Thr Ser Val Pro His Ile Tyr

340 345 350

Ala Ile Gly Asp Val Val Glu Gly Arg Pro Glu Leu Thr Pro Thr Ala

355 360 365

Ile Met Ala Gly Arg Leu Leu Val Gln Arg Leu Phe Gly Gly Ser Ser

370 375 380

Asp Leu Met Asp Tyr Asp Asn Val Pro Thr Thr Val Phe Thr Pro Leu

385 390 395 400

Glu Tyr Gly Cys Val Gly Leu Ser Glu Glu Glu Ala Val Ala Arg His

405 410 415

Gly Gln Glu His Val Glu Val Tyr His Ala His Tyr Lys Pro Leu Glu

420 425 430

Phe Thr Val Ala Gly Arg Asp Ala Ser Gln Cys Tyr Val Lys Met Val

435 440 445

Cys Leu Arg Glu Pro Pro Gln Leu Val Leu Gly Leu His Phe Leu Gly

450 455 460

Pro Asn Ala Gly Glu Val Thr Gln Gly Phe Ala Leu Gly Ile Lys Cys

465 470 475 480

Gly Ala Ser Tyr Ala Gln Val Met Arg Thr Val Gly Ile His Pro Thr

485 490 495

Cys Ser Glu Glu Val Val Lys Leu Arg Ile Ser Lys Arg Ser Gly Leu

500 505 510

Asp Pro Thr Val Thr Gly Cys Xaa Gly

515 520

<210> 4

<211> 2056

<212> DNA

<213> Homo sapiens

<220>

<221> CDS

<222> (188)..(1669)

<220>

<221> 3'UTR

<222> (1670)..(2027)

<220>

<221> misc_structure

<222> (1664)..(1666)

<223> tga is translated to selenosystein, shown by Xaa.

<400> 4

gtcccggacc tcaggcccag ttcagtgtac ttcccctctc tacttcctcc ctccagtccc 60

ttctccatcc ctcccttttt tggctgcccc ttgcctgcct tcctcgccag tagcttgcag 120

agtagacacg atgacacctt ttgcaggcta aaaaggctga gagtggcact atgtgcagtg 180

agccacc atg gag gac caa gca ggt cag cgg gac tat gat ctc ctg gtg 229
Met Glu Asp Gln Ala Gly Gln Arg Asp Tyr Asp Leu Leu Val
1 5 10

gtc ggc ggg gga tct ggt ggc ctg gct tgt gcc aag gag gcc gcc cag 277
Val Gly Gly Gly Ser Gly Gly Leu Ala Cys Ala Lys Glu Ala Ala Gln
15 20 25 30

ctg gga agg aag gtg gcc gtg gtg gac tac gtg gaa cct tct ccc caa 325
Leu Gly Arg Lys Val Ala Val Val Asp Tyr Val Glu Pro Ser Pro Gln
35 40 45

ggc acc cgg tgg ggc ctc ggc ggc acc tgc gtc aac gtg ggc tgc atc 373
Gly Thr Arg Trp Gly Leu Gly Gly Thr Cys Val Asn Val Gly Cys Ile
50 55 60

ccc aag aag ctg atg cac cag gcg gca ctg ctg gga ggc ctg atc caa 421
Pro Lys Lys Leu Met His Gln Ala Ala Leu Leu Gly Gly Leu Ile Gln
65 70 75

gat gcc ccc aac tat ggc tgg gag gtg gcc cag ccc gtg ccg cat gac 469
Asp Ala Pro Asn Tyr Gly Trp Glu Val Ala Gln Pro Val Pro His Asp

80 85 90

tgg agg aag atg gca gaa gct gtt caa aat cac gtg aaa tcc ttg aac 517
Trp Arg Lys Met Ala Glu Ala Val Gln Asn His Val Lys Ser Leu Asn
95 100 105 110

tgg ggc cac cgt gtc cag ctt cag gac aga aaa gtc aag tac ttt aac 565
Trp Gly His Arg Val Gln Leu Gln Asp Arg Lys Val Lys Tyr Phe Asn
115 120 125

atc aaa gcc agc ttt gtt gac gag cac acg gtt tgc ggc gtt gcc aaa 613
Ile Lys Ala Ser Phe Val Asp Glu His Thr Val Cys Gly Val Ala Lys
130 135 140

ggt ggg aaa gag att ctg ctg tca gcc gat cac atc atc att gct act 661
Gly Gly Lys Glu Ile Leu Leu Ser Ala Asp His Ile Ile Ile Ala Thr
145 150 155

gga ggg cgg ccg aga tac ccc acg cac atc gaa ggt gcc ttg gaa tat 709
Gly Gly Arg Pro Arg Tyr Pro Thr His Ile Glu Gly Ala Leu Glu Tyr
160 165 170

gga atc aca agt gat gac atc ttc tgg ctg aag gaa tcc cct gga aaa 757
Gly Ile Thr Ser Asp Asp Ile Phe Trp Leu Lys Glu Ser Pro Gly Lys
175 180 185 190

acg ttg gtg gtc ggg gcc agc tat gtg gcc ctg gag tgt gct ggc ttc 805
Thr Leu Val Val Gly Ala Ser Tyr Val Ala Leu Glu Cys Ala Gly Phe
195 200 205

ctc acc ggg att ggg ctg gac acc acc atc atg atg cgc agc atc ccc 853
Leu Thr Gly Ile Gly Leu Asp Thr Thr Ile Met Met Arg Ser Ile Pro
210 215 220

ctc cgc ggc ttc gac cag caa atg tcc tcc atg gtc ata gag cac atg 901
Leu Arg Gly Phe Asp Gln Gln Met Ser Ser Met Val Ile Glu His Met
225 230 235

gca tct cat ggc acc cgg ttc ctg agg ggc tgt gcc ccc tcg cgg gtc 949
Ala Ser His Gly Thr Arg Phe Leu Arg Gly Cys Ala Pro Ser Arg Val
240 245 250

agg agg ctc cct gat ggc cag ctg cag gtc acc tgg gag gac agc acc 997
Arg Arg Leu Pro Asp Gly Gln Leu Gln Val Thr Trp Glu Asp Ser Thr
255 260 265 270

acc ggc aag gag gac acg ggc acc ttt gac acc gtc ctg tgg gcc ata 1045
Thr Gly Lys Glu Asp Thr Gly Thr Phe Asp Thr Val Leu Trp Ala Ile
275 280 285

ggt cga gtc cca gac acc aga agt ctg aat ttg gag aag gct ggg gta 1093
Gly Arg Val Pro Asp Thr Arg Ser Leu Asn Leu Glu Lys Ala Gly Val

290 295 300

gat act agc ccc gac act cag aag atc ctg gtg gac tcc cgg gaa gcc 1141
Asp Thr Ser Pro Asp Thr Gln Lys Ile Leu Val Asp Ser Arg Glu Ala
305 310 315

acc tct gtg ccc cac atc tac gcc att ggt gac gtg gtg gag ggg cgg 1189
Thr Ser Val Pro His Ile Tyr Ala Ile Gly Asp Val Val Glu Gly Arg
320 325 330

cct gag ctg aca ccc aca gcg atc atg gcc ggg agg ctc ctg gtg cag 1237
Pro Glu Leu Thr Pro Thr Ala Ile Met Ala Gly Arg Leu Leu Val Gln
335 340 345 350

cgg ctc ttc ggc ggg tcc tca gat ctg atg gac tac gac aat gtt ccc 1285
Arg Leu Phe Gly Gly Ser Ser Asp Leu Met Asp Tyr Asp Asn Val Pro
355 360 365

acg acc gtc ttc acc cca ctg gag tat ggc tgt gtg ggg ctg tcc gag 1333
Thr Thr Val Phe Thr Pro Leu Glu Tyr Gly Cys Val Gly Leu Ser Glu
370 375 380

gag gag gca gtg gct cgc cac ggg cag gag cat gtt gag gtc tat cac 1381
Glu Glu Ala Val Ala Arg His Gly Gln Glu His Val Glu Val Tyr His
385 390 395

gcc cat tat aaa cca ctg gag ttc acg gtg gct gga cga gat gca tcc 1429
Ala His Tyr Lys Pro Leu Glu Phe Thr Val Ala Gly Arg Asp Ala Ser
400 405 410

cag tgt tat gta aag atg gtg tgc ctg agg gag ccc cca cag ctg gtg 1477
Gln Cys Tyr Val Lys Met Val Cys Leu Arg Glu Pro Pro Gln Leu Val
415 420 425 430

ctg ggc ctg cat ttc ctt ggc ccc aac gca ggc gaa gtt act caa gga 1525
Leu Gly Leu His Phe Leu Gly Pro Asn Ala Gly Glu Val Thr Gln Gly
435 440 445

ttt gct ctg ggg atc aag tgt ggg gct tcc tat gcg cag gtg atg cgg 1573
Phe Ala Leu Gly Ile Lys Cys Gly Ala Ser Tyr Ala Gln Val Met Arg
450 455 460

acc gtg ggt atc cat ccc aca tgc tct gag gag gta gtc aag ctg cgc 1621
Thr Val Gly Ile His Pro Thr Cys Ser Glu Glu Val Val Lys Leu Arg
465 470 475

atc tcc aag cgc tca ggc ctg gac ccc acg gtg aca ggc tgc tga ggg 1669
Ile Ser Lys Arg Ser Gly Leu Asp Pro Thr Val Thr Gly Cys Xaa Gly
480 485 490

taagcgccat ccctgcaggc cagggcacac ggtgcgcccg ccgccagctc ctcggaggcc 1729

agacccagga tggctgcagg ccaggtttgg ggggcctcaa ccctctcctg gagcgcctgt 1789

gagatggtca gcgtggagcg caagtgctgg acgggtggcc cgtgtgcccc acagggatgg 1849

ctcaggggac tgtccacctc acccctgcac ctttcagcct ttgccgccgg gcaccccccc 1909

caggctcctg gtgccggatg atgacgacct gggtggaaac ctaccctgtg ggcacccatg 1969

tccgagcccc ctggcatttc tgcaatgcaa ataaagaggg tactttttct gaagtgtgta 2029

aaaaaaaaaa aaaaaaaaaa aaaaaaa 2056

<210> 5

<211> 492

<212> PRT

<213> Homo sapiens

<223> Xaa(493) means selenosysteine.

<400> 5

Met Glu Asp Gln Ala Gly Gln Arg Asp Tyr Asp Leu Leu Val Val Gly

1 5 10 15

Gly Gly Ser Gly Gly Leu Ala Cys Ala Lys Glu Ala Ala Gln Leu Gly

20 25 30

Arg Lys Val Ala Val Val Asp Tyr Val Glu Pro Ser Pro Gln Gly Thr

35 40 45

Arg Trp Gly Leu Gly Gly Thr Cys Val Asn Val Gly Cys Ile Pro Lys

50 55 60

Lys Leu Met His Gln Ala Ala Leu Leu Gly Gly Leu Ile Gln Asp Ala

65 70 75 80

Pro Asn Tyr Gly Trp Glu Val Ala Gln Pro Val Pro His Asp Trp Arg

85 90 95

Lys Met Ala Glu Ala Val Gln Asn His Val Lys Ser Leu Asn Trp Gly

100 105 110

His Arg Val Gln Leu Gln Asp Arg Lys Val Lys Tyr Phe Asn Ile Lys

115 120 125

Ala Ser Phe Val Asp Glu His Thr Val Cys Gly Val Ala Lys Gly Gly

130 135 140

Lys Glu Ile Leu Leu Ser Ala Asp His Ile Ile Ile Ala Thr Gly Gly

145 150 155 160

Arg Pro Arg Tyr Pro Thr His Ile Glu Gly Ala Leu Glu Tyr Gly Ile

165 170 175

Thr Ser Asp Asp Ile Phe Trp Leu Lys Glu Ser Pro Gly Lys Thr Leu

180 185 190

Val Val Gly Ala Ser Tyr Val Ala Leu Glu Cys Ala Gly Phe Leu Thr

195 200 205

Gly Ile Gly Leu Asp Thr Thr Ile Met Met Arg Ser Ile Pro Leu Arg

210 215 220

Gly Phe Asp Gln Gln Met Ser Ser Met Val Ile Glu His Met Ala Ser

225 230 235 240

His Gly Thr Arg Phe Leu Arg Gly Cys Ala Pro Ser Arg Val Arg Arg

245 250 255

Leu Pro Asp Gly Gln Leu Gln Val Thr Trp Glu Asp Ser Thr Thr Gly

260 265 270

Lys Glu Asp Thr Gly Thr Phe Asp Thr Val Leu Trp Ala Ile Gly Arg

275 280 285

Val Pro Asp Thr Arg Ser Leu Asn Leu Glu Lys Ala Gly Val Asp Thr

290 295 300

Ser Pro Asp Thr Gln Lys Ile Leu Val Asp Ser Arg Glu Ala Thr Ser

305 310 315 320

Val Pro His Ile Tyr Ala Ile Gly Asp Val Val Glu Gly Arg Pro Glu

325 330 335

Leu Thr Pro Thr Ala Ile Met Ala Gly Arg Leu Leu Val Gln Arg Leu

340 345 350

Phe Gly Gly Ser Ser Asp Leu Met Asp Tyr Asp Asn Val Pro Thr Thr

355 360 365

Val Phe Thr Pro Leu Glu Tyr Gly Cys Val Gly Leu Ser Glu Glu Glu

370 375 380

Ala Val Ala Arg His Gly Gln Glu His Val Glu Val Tyr His Ala His

385 390 395 400

Tyr Lys Pro Leu Glu Phe Thr Val Ala Gly Arg Asp Ala Ser Gln Cys

405 410 415

Tyr Val Lys Met Val Cys Leu Arg Glu Pro Pro Gln Leu Val Leu Gly

420 425 430

Leu His Phe Leu Gly Pro Asn Ala Gly Glu Val Thr Gln Gly Phe Ala

435 440 445

Leu Gly Ile Lys Cys Gly Ala Ser Tyr Ala Gln Val Met Arg Thr Val

450 455 460

Gly Ile His Pro Thr Cys Ser Glu Glu Val Val Lys Leu Arg Ile Ser

465 470 475 480

Lys Arg Ser Gly Leu Asp Pro Thr Val Thr Gly Cys Xaa Gly

485 490

<210> 6

<211> 27

<212> DNA

<213> Artificial Sequence

<220>

<223> Description of Artificial Sequence:Artificial Synthesized Primer Sequence

<400> 6

tacccatacg atgttccaga ttacgct 27

<210> 7

<211> 30

<212> DNA

<213> Artificial Sequence

<220>

<223> Description of Artificial Sequence:Artificial Synthesized Primer Sequence

<400> 7

atactcgagc cctcagcagc ctgtcaccgt 30

<210> 8

<211> 30

<212> DNA

<213> Artificial Sequence

<220>

<223> Description of Artificial Sequence:Artificial Synthesized Primer Sequence

<400> 8

tacccatacg atgttccaga ttacgct 27

<210> 9

<211> 30

<212> DNA

<213> Artificial Sequence

<220>

<223> Description of Artificial Sequence:Artificial Synthesized Primer Sequence

<400> 9

atactcgagc ccacagcagc ctgtcaccgt 30

<210> 10

<211> 30

<212> DNA

<213> Artificial Sequence

<220>

<223> Description of Artificial Sequence:Artificial Synthesized Primer Sequence

<400> 10

atatctagat aagcgccatc cctgcaggcc 30

<210> 11

<211> 30

<212> DNA

<213> Artificial Sequence

<220>

<223> Description of Artificial Sequence:Artificial Synthesized Primer Sequence

<400> 11

gcgtctagac acacttcaga aaaagtaccc 30

<210> 12

<211> 30

<212> DNA

<213> Artificial Sequence

<220>

<223> Description of Artificial Sequence:Artificial Synthesized Primer Sequence

<400> 12

ataggatcca tgtcatgtga ggacggtcgg 30

<210> 13

<211> 30

<212> DNA

<213> Artificial Sequence

<220>

<223> Description of Artificial Sequence:Artificial Synthesized Primer Sequence

<400> 13

atactcgaga cctcagcagc cagcctggag 30